## Europäisches Patentamt

## European Patent Office

## Office européen des brevets

(11) Publication number: **0 137 234**
**B1**

(12) **EUROPEAN PATENT SPECIFICATION**

(45) Date of publication of patent specification: **10.10.90**

(21) Application number: **84109773.6**

(22) Date of filing: **16.08.84**

(51) Int. Cl.5: **A 61 K 39/395, A 61 K 37/24, A 61 K 39/00 // C12P21/00, C12N15/00**

(54) Physiologically active compositions.

(30) Priority: **17.08.83 GB 8322115**
**25.08.83 GB 8322826**

(43) Date of publication of application:
**17.04.85 Bulletin 85/16**

(45) Publication of the grant of the patent:
**10.10.90 Bulletin 90/41**

(84) Designated Contracting States:
**AT BE CH DE FR GB IT LI LU NL SE**

(56) References cited:

CHEMICAL ABSTRACTS, vol. 98, no. 7, 14th February 1983, page 497, abstract no. 51671a, Columbus, Ohio, US; J. IVANVI: "Study of antigenic structure and inhibition of activity of human growth hormone and chorionic somatomam motropin by monoclonal antibodies"

(73) Proprietor: **THE WELLCOME FOUNDATION LIMITED**
**Unicorn House 160 Euston Road**
**London NW1 2BP (GB)**

(72) Inventor: **Aston, Roger**
**Langley Court**
**Beckenham Kent BR3 3BS (GB)**
Inventor: **Holder, Andrew Thomas**
**30 Guildford Street**
**London WC1N 1EH (GB)**
Inventor: **Ivanyi, Juraj**
**Langley Court**
**Beckenham Kent BR3 3BS (GB)**

(74) Representative: **Sandmair, Kurt, Dr. et al**
**Patentanwälte Schwabe, Sandmair, Marx**
**Stuntzstrasse 16**
**D-8000 München 80 (DE)**

(56) References cited:
CHEMICAL ABSTRACTS, vol. 97, no. 5, 2nd August 1982, pages 57,58, abstract no. 33510x, Columbus, Ohio, US; L.A. RETEGUI et al.: "Monoclonal antibodies against growth hormone: effects on the hormone interaction with specific cell surface receptors"

# EP 0 137 234 B1

**Description**

This invention relates to hormone activity in vertebrate species.

The term "hormone" has been defined as encompassing any substance released from one part of the body and acting selectively on at least one other, distant, part. Many such substances are secreted by one of the endocrine glands, an example being the secretion of growth hormone from the pituitary gland. The basis for the selectivity for a given target tissue or tissues is the presence or absence in the tissue of receptors which bind the substance specifically. In the case of the protein hormones, such as insulin, the receptors are on the cell surface. Interferon has been implicated in the defence of mammalian cells against viral attack. It is not secreted by an endocrine gland and does not have the specificity of target tissue that, say, growth hormone has. Nevertheless, it is a large glycosylated protein molecule and binds to specific cell surface receptors. There are also certain similar substances which influence the activity of cell by binding to specific receptors on the cell surface, for example tumour necrosis factor and lymphokines such as interleukins. In this specification, the term "hormone" is used to embrace all such protein or polypeptide entities (optionally glycosylated) and the like having a cell-surface receptor.

It has been shown that certain antibodies against insulin and against epidermal growth factor (EGF) potentiate or mimic the activity of those hormones *in vitro* (Y. Schechter *et al*, Proc. Nat. Acad. Sic. 76(6), 2720, (1979) and Y. Schechter *et al*, Nature *278, 835*, (1979)). It is thought that this occurs because the antibodies, being bivalent, cause an aggregation of the hormone-receptor complexes on the cell-surface, such aggregation being involved in activating the second messenger within the cell; monovalent Fab fragments of antibodies do not cause such potentiation or mimicry. These studies have solely been concerned with characterising the insulin and EGF receptors and in identifying the mode of action of such hormones. Furthermore, not all such studies have demonstrated such potentiation (de Pirro *et al*, Diabetologia *19*, 118 (1980) and Schechter *et al*, Proc. Nat. Acad. Sci, 75(12), 5788 (1978)). There has been no suggestion that such potentiation would occur *in vivo* or that the phenomenon is more widely applicable. Indeed, the generation of antibodies against insulin and other hormones *in vivo* was thought to be highly undesirable since the hormone-antibody complexes would be cleared by the body's immune system and, far from being potentiated, the action of the hormone would be negated—see for example Schwartz J., Endocrinology, 107(4), 877; Fraisier, Endocrine Reviews *4(2)*, 155 and Gause *et al*, Endocrinology 112(5), 1559 on growth hormone, and Blake & Kelch, Endocrinology, 109(6), 2175 on luteinising hormone releasing hormone.

It has now surprisingly been found that the administration of certain specific antibodies to hormones can potentiate or mimic the activity of the hormone, provided that the epitope specificity of the antibody is chosen appropriately.

Accordingly, one aspect of the present invention provides a formulation comprising antibodies to a hormone, the epitope specificity of at least some of the antibodies being so chosen that the formulation will potentiate or mimic, when administered to a vertebrate, the administration of the hormone in that vertebrate.

A second aspect of the invention provides a formulation comprising complexes of (a) a hormone and (b) at leat one type of antibody to that hormone, the epitope specificity of at least some of the antibodies being so chosen that the formulation will potentiate or mimic, when administered to a vertebrate, the administration of the hormone in that vertebrate.

A third aspect of the invention provides a method of potentiating or mimicking hormone administration in a "normal" vertebrate (as herein defined) by administering to the vertebrate a formulation comprising antibodies to the hormone, the epitope specificity of at least some of the antibodies being so chosen that the formulation will potentiate or mimic, when administered to a vertebrate, the administration of the hormone in that vertebrate.

The term "normal" is used herein to indicate an individual having sufficient endogenous amount of the hormone in question for normal functioning of the tissues regulated by that hormone.

A fourth aspect of the invention provides a method of potentiating or mimicking hormone administration in a "normal" vertebrate (as herein defined) by administering to the vertebrate a formulation comprising complexes of (a) the hormone and (b) at least one type of antibody to that hormone, the epitope specificity of at least some of the antibodies being so chosen that the formulation will potentiate or mimic, when administered to the vertebrate, the administration of the hormone in that vertebrate.

A fifth aspect provides a method of treating a human or other vertebrate having abnormally low hormone-regulated tissue function by administering to the vertebrate a pharmaceutical formulation comprising antibodies to the hormone in question, the epitope specificity of at least some or the antibodies being so chosen that the formulation will potentiate or minic, when administered to the vertebrate, the administration of the hormone in that vertebrate.

A sixth aspect provides a method of treating a vertebrate having an abnormally low hormone-regulated tissue function by administering to the human or animal a pharmaceutical formulation comprising complexes of (a) the hormone in question and (b) at least one type of antibody to that hormone, the epitope specificity of at least some of the antibodies being so chosen that the formulation will potentiate or mimic, when administered to a vertebrate, the administration of the hormone in that vertebrate.

The potentiation of mimicry of the administration of growth hormone, insulin, thyroid stimulating hormone and interferon are particularly preferred aspects of the invention.

The clinical abnormalities which result from a deficiency of a given hormone are in many cases well characterised and will not be listed here. However, examples include (from a deficiency of growth hormone) pituitary dwarfism. Turner's syndrome and cachexia, (from a deficiency of insulin) diabetes and (from a deficiency of thyroid stimulating hormone) cretinism, simple goitre and myxedema.

By antibody "to" a particular hormone, we mean an antibody which will bind to that hormone. Thus, the antibody need not have been created in response to that specific hormone. For example several antibodies raised against growth hormone (GH) will cross-react with chorionic somatomammotropin (CS) because of the extensive sequence homology between the two hormones. Furthermore, it may be possible to raise antibodies to a synthetic analogue or hormone of a portion of it.

It is to be noted that the antibodies need not necessarily be to the specific hormone of the species to which the formulation or method of the invention is being applied. Preferably, however, they are. It has been found that not all antibodies to the hormone will potentiate or mimic the administration of that hormone; instead, the ability of an antibody to act in accordance with the invention appears to depend on the specific determinant (i.e. antigenic site) for the antibody on the hormone. It will therefore readily be appreciated that polyclonal antibodies (that is to say, a collection of antibodies having a range of determinant specificities) are less suitable for use in formulations or methods in accordance with the invention, than are monoclonal antibodies. The man skilled in the art will readily, having read this specification, be able by means of routine experimentation to select a monoclonal antibody effective in carrying out the invention. Mixtures of suitable monoclonal antibodies may in some circumstances be used. However, it is nevertheless possible to use animal or human antisera raised by 'conventional' immunization provided that the epitope specificity of the antibodies is as described. Particularly preferred monoclonal antibodies for growth hormone (GH) and chorionic somatomammotropin (CS) are EB01 and EB02. Antibody QB01 is preferred for prolactin (PRL).

It has been found that the presence of the hormone in the animal is necessary for the antibody (when administered alone) to act in the manner described. Thus, in the case of "normal" individuals, administration of the selected antibody alone will have the described effect but, for example, in individuals without endogenous GH, such as pituitary dwarf human children, GH must be administered as well as, but not necessarily simultaneously with, the antibody.

Instead of preparing the antibody outside the animal, it is possible to raise antibodies of the appropriate specificity by injecting the animal with a preselected fragment of a suitable growth hormone molecule in combination with an adjuvant. The fragment will be so chosen as to comprise only the epitope or epitopes to which one or more of the hormone-potentiating antibodies are specific and may be derived by cleaving the hormone appropriately, or by synthesising a peptide fragment (or an analogue to such a fragment). By choosing portions of the hormone rich in hydrophilic residues, one is more likely to be creating or selecting a fragment (an "antigenic determinant") which is on the surface of the complete hormone molecule and which will therefore cross-react with the complete molecule. Equally, the fragment should not contain the site of the hormone which binds to the cell-surface receptor, nor any site which binds a third agent which causes a conformational change such that receptor binding is inhibited. Otherwise, the antibodies produced are likely to inhibit, rather than potentiate, the action of the hormone. Thus, in a successful immunisation of this type, a polyclonal collection of antibodies of narrow specificity is created within the animal in question, thus enabling less frequent injection of the animal than would be the case if exogenous antibodies were passively administered. It will be appreciated that, instead of an actual fragment, a functional fragment could be used, in which the undesirable epitopes of the molecule are present but are shielded from antibody access in some way. The term "fragment" is used in this specification to cover actual and functional fragments.

Accordingly, the present invention also provides a method of increasing a hormone-regulated response of a vertebrate by administering to the vertebrate a preparation comprising at least one pre-selected "fragment" (as herein defined) of an appropriate hormone, optionally in combination with an adjuvant.

The invention also encompasses such a preparation and methods of making such a preparation by conventional means. In such conventional vaccines, immunological carriers are frequently used to enhance the immunogenicity of the antigen, for example keyhole limpet haemocyanin or tetanus toxoid. Similarly, adjuvants are often included to stimulate the immune system, for example aluminium hydroxide, saponin or muramyl dipeptide. Generally, about 0.001 to 10 μmoles of antigen should be present in a unit dose, preferably about 0.01 to 0.05 μmoles, although the selection of a suitable amount of the antigen is well within the capabilities of one skilled in the art.

In the case of passive transfer of antibodies to a vertebrate, approximately $10^4$—$10^7$, preferably $10^5$—$10^6$ $ABT_{50}$ units of antibodies should be administered in any suitable sterile medium, such as saline, to give a dose of 0.01 to 10 ml, preferably about 0.5 ml.

To take only three hormones as an example, namely GH, CS and PRL, formulations or methods in

3

accordance with the invention are believed to offer potential in:

(a) accelerating the attainment of full growth of industrially important (i.e. farmed) animals such as cattle, pigs and poultry or achieving such growth on reduced amounts of feed;

(b) increasing the growth of such animals beyond the normal maximum;

(c) increasing the duration or extent of lactation in mammals, for example to obtain a greater milk yield from cattle or to enable a human mother to breast-feed an infant;

(d) increasing the proportion of lean meat to fat in farmed animals;

(e) increasing the growth of fleece, fur or other useful surface products of animals, for example sheep;

(f) treating a GH-deficient individual, for example a dwarf child, to enable normal growth to occur.

In all cases, it is believed that the use of formulations and/or methods in accordance with the invention may offer significant cost-saving and labour-saving advantages in comparison with the use of the hormone alone, not least because the potentiation of the hormone action is expected to result in fewer administrations being needed. Furthermore, a reduction of possibly harmful residues in the meat or milk of treated animals may be expected. Finally, because farmed animals are frequently routinely injected with other vaccines, for example against foot and mouth disease, it would be extremely convenient to incorporate in such a vaccine a formulation in accordance with the present invention.

The invention will now be described by way of the following non-limiting Examples.

Abbreviations

List of abbreviations

| | |
|---|---|
| hGH | human growth hormone |
| hCS | human chorionic somatomammotropin |
| hPRL | human prolactin |
| bGH | bovine growth hormone |
| MAB | monoclonal antibody |
| PBS | phosphate-buffered saline |
| PMSF | phenyl methyl sulphonyl fluoride |
| Ig | immunoglobulin |
| MHC | major histocompatibility complex |
| SPRIA | solid phase radioimmunoassay |
| $W_{to}$ | weight at time 0 |
| SDS-PAGE | SDS polyacrylamide gel electrophoresis |
| EDTA | ethylene diamine tetracetic acid |
| RIA | radio-immuno assay |

Statistical evaluation

Arithmetic means and standard deviation values were calculated using conventional methods. Differences between groups were assessed by unpaired Student's t-test.

Description of Figures

Figure 1 relates to Example A and shows weight gain in dwarf mice with compositions in accordance with the invention;

Figure 2 relates to Example C and shows corresponding weight gain in normal mice;

Figure 3 relates to Example D and shows the weight gain of the pigeon crop sac; and

Figure 4 relates to Example E and shows the weight gain of marmosets.

Preparative Examples

Example 1

Preparation of monoclonal antibody to human growth hormone (hGH)

The antibodies employed in this study are available from Wellcome Diagnostics, Temple Hill, Dartford, Kent, U.K. and have been characterized extensively (Ivanyi, 1982 a b, Aston and Ivanyo, 1983). BALB/c mouse spleen cells were fused with NSI myeloma cells and cloned by standard techniques (Ivanyi and Davis, 1980, 1981). The antibodies derived were all of the $IgG_1$ isotype and were non-precipitating when examined by double diffusion in agar. Four determinants have been defined on hGH by competition assays (QA68, NA71, EB01 and EB02) of which two are completely shared with hCS (EB01 and EB02). However, none of the antibodies cross-reacted with human prolactin. Antibody concentrations have been expressed as $ABT_{50}$ values which correspond to the reciprocal antibody titre required to give 50 percent binding of $^{125}I$-hGH by RIA (Ivanyi, 1982a). Binding studies with proteolytically modified forms of hGH suggest that all four determinants are located in the first 1—139 residues with the EB01 determinant also represented in the sequence region 146—191 (Aston and Ivanyi, 1983).

Example 2

Preparation of Fab′ fragment of EB01

Ascitic globulin (5 mg/ml) of EB01 was affinity purified on hGH (100 mg) immobilized on CNBr- activated Sepharose. Retained material was eluted with glycine-HCl buffer pH 2.3 and tubes containing protein material were immediately adjusted to pH 7.5 with NaOH (1 M). The purified antibody was concentrated to 20 mg/ml (2 ml) and dialysed against sodium phosphate buffer (0.5 M, pH 8.0) containing cysteine (0.01 M) and EDTA (.002 M).

This material was digested with 0.4 mg of papain (BDH) for 4 hours at 37°C followed by dialysis against PBS to remove the cysteine and EDTA.

Subsequently, the dialysate was applied to a column of DEAE cellulose (20 cm×1.2 cm) and eluted with a linear gradient consisting of sodium phosphate buffer (0.005 M—0.3 M, pH 8.0). The first peak to be eluted from the column contained no antibody heavy chain as determined by SDS-PAGE and retained an activity of $10^{-3} \times ABT_{50}$.

Example 3
Hormones

Human growth hormone employed for injection was derived from stocks of outdated clinical grade material obtained by special agreement with the Institute of Child Health, London, whereas hormone used in assays was of >99% purity. hGH is available from RIA (UK) Ltd., Washington, Co. Durham U.K. Radioiodination of hGH was performed with lactoperoxidase resulting in a tracer of high specific activity ($80 \times 10^6$ cpm/g) (Linde et al, 1981). Monomeric $^{125}I$-hGH was separated from any aggregated material prior to assay by Ultrogel column chromatography. Ultrogel is a trade mark of LKB Ltd., Cambridge, U.K. Antibody-hormone complexes for administration into animals were prepared by mixing the solutions for 1 hour prior to injection. In chronic experiments, where injections were given for several weeks, the complexes were prepared in batches enough for 1 week and stored at ±4°C.

A soluble extract was prepared from the marmoset pituitary gland by homogenizing the tissue in 0.05 M sodium bicarbonate 2 mM PMSF pH 8.6. The resulting homogenate was centrifuged at 10,000 g for 20 minutes and the supernatant (20 ml) was tested.

Biological Examples
Example A
Cumulative weight gains in hGH-EB01 complex treated dwarf mice

Dwarf mice were bred from normal animals heterozygous for the dw gene or from a heterozygous female mouse and a male homozygous dwarf mouse treated with thyroxine. The dwarf mice, weighing 9.1±0.4 g, were allocated at random to treatment groups of six animals and then distributed among several small cages each containing one representative of each treatment group. Hormones were injected subcutaneously in the back in 0.1 ml, for the periods indicated. Weights were measured at the onset, during and sometimes after treatment. Weight gains in short-term experiments or the cumulative weight gains over several days of study were expressed as relative values (%) related to initial whole body weights or as net weights (g). Tail lengths were measured by the method of Hughes and Tanner (1970).

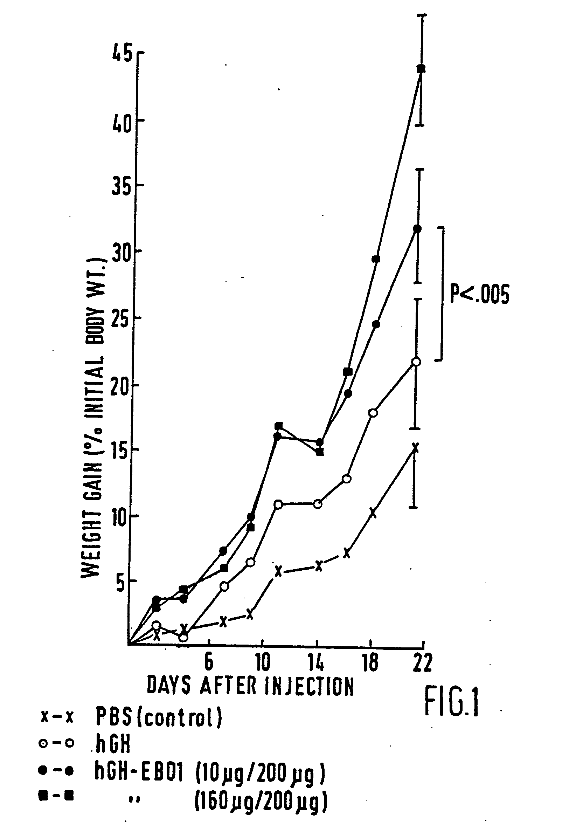

Over a three week period, control mice, treated with phosphate-buffered saline (PBS), increased their relative weight gains by about 15% (Figure 1). Mice treated with 10 μg hGH gained 22% over starting weight over the same period. However, hGH-EB01 complexes raised the cumulative weight gain to 34%, which corresponds to an additional 12% increment over that achieved from treatment with hGH only.

Raising the hGH dose in the complex from 10 μg to 160 μg, increased the weight gains to 44% of the initial body weight. It is apparent (Figure 1) that the differences between treated groups and controls progressively increased over the 21 day test period. Whilst hGH-MAB complexes produced a significant increment within 48 h, the difference between the group treated with hGH only and the PBS-injected control group was not apparent until day 7.

Example B
Growth potentiation by EB01 Fab' fragments

In order to assess whether the bivalency of EB01 antibody was a pre-requisite for growth potentiation, complexes of EB01-Fab'-hGH were examined for their effects on $^{35}SO_4^{2-}$ uptake in dwarf mice.

Dwarf mice within a relatively narrow weight range (7—10 g) were randomized by use of tables of random numbers (Fisher and Yates, 1957) and injected with a dose of $^{35}SO_4^{2-}$ related to body weight (0.5 Ci/g body weight) 24 h after the final hormone injection (Herbai, 1970). Mice were killed 20 h later when rib cages were removed, placed in boiling water for 20 min, soaked overnight in saturated sodium sulphate and washed in tap water for 2 h and distilled water for 1 h. The bony portion of ribs together with about 1 mm of adjacent rib cartilage (costochondral junction) was then cut away leaving the costal cartilages attached to the sternum. All adhering soft tissue was removed and the five longest costal cartilages which articulated directly with the sternum were detached whole from each side of the rib cage and combined for each animal. Each pool of ten costal cartilages was then dried at room temperature overnight, weighed and processed for the measurement of $^{35}SO_4^{2-}$. The uptake of $^{35}SO_4^{2-}$ by costal cartilage was expressed as disintegrations per minute per mg of cartilage.

The levels of sulphate uptake by cartilage potentiated by EB01 or EB01-Fab' were not significantly different when comparing the antibody and fragments at the same $ABT_{50}$ dose (Table 1). However, the growth observed in the presence of hGH alone was significantly less than that observed for Fab'-hGH.

Increasing the valency of the Fab' fragment by including a "second" either monoclonal anti-light chain antibody (TC187) or polyclonal anti-mouse Ig antibody did not significantly alter the $^{35}SO_4{}^{2-}$ uptake. Furthermore, Fab' fragments did not competitively inhibit the potentiating effect of EB01 antibody. Preparation of complexes of EB01 with equimolar quantities of hGH and hCS also did not increase the degree of potentiation. Such complexes would comprise mainly the species hGH-EB01-hCS which would be expected to have decreased activity if bivalency was necessary, since hCS has only 10% somatotropic activity of hGH. Indeed, the EB01-hCS complex resulted in significantly lower growth activity than the corresponding complex with hGH.

TABLE 1

The effect of antibody Fab fragment-hGH complex on sulphate uptake activity

| Hormone (µg) | Antibody (µg) | | $^{35}SO_4{}^{2-}$ uptake dpm/mg±SD |
|---|---|---|---|
| | EB01 | Anti mouse Ig | |
| hGH(160) | — | — | 1640±160 |
| hGH(160) | Ig(200) | — | 4280±300 |
| hGH(160) | Ig(20) | — | 2500±400 |
| hGH(160) | Fab(20) | — | 2700±120* |
| hGH(160) | Fab(20) | TC187(12) | 2300±100 |
| hGH(160) | Fab(20) | R-Poly(5) | 2000±100 |
| hGH(160) | Fab(20)+Ig(200) | — | 4880±190 |
| hCS(160) | Ig(200) | — | 1200±70 |
| hGH(80)+hCS(80) | Ig(200) | — | 3820±250 |
| hGH(80)+hCS(80) | Ig(200) | R-Poly(5) | 4250±500 |
| PBS | — | — | 600±75 |

Dwarf mice (n=6) were injected twice with hormone plus antibody (0 and 24 h) followed by $^{35}SO_4{}^{2-}$ (48 h) and harvested at 72 h.

TC187=Rat monoclonal anti-mouse L-chain; R-Poly=rabbit polyclonal anti-mouse Ig.

*p<.001 compared with hGH alone.

Example C

Potentiation of growth in juvenile BALB/c mice

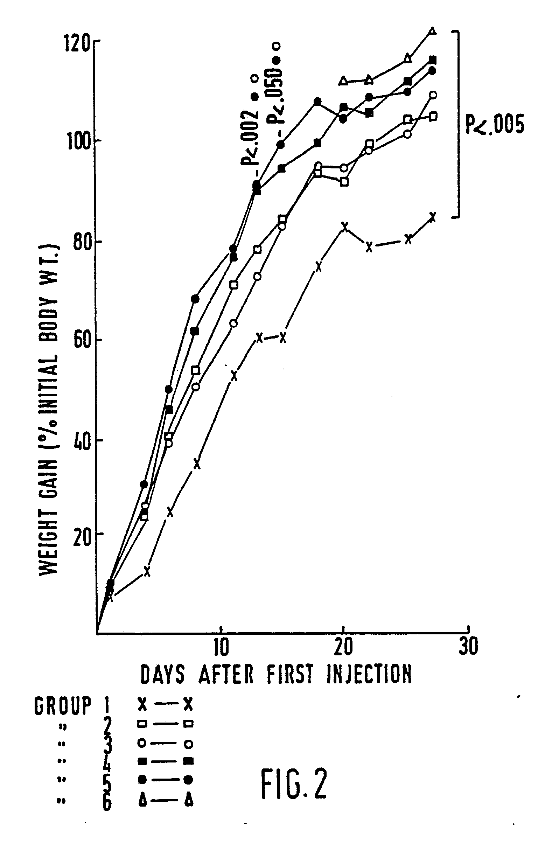

Since the stimulation of growth in dwarf mice is exercised over a background of very slow activity, it was of interest to ascertain if the antibody mediated potentiation effect could be demonstrated in normally, i.e. rapidly, growing juvenile mice. Three weeks old BALB/c mice weighing between 7—10 g were randomized and injected as described above for dwarf mice. Weights were taken 3 times/week in chronic experiments. Group 1 received PBS twice weekly throughout; Group 2 had 160 µg hGH three times over 1 week and Group 3 the same thrice weekly for 4 weeks; Groups 4 and 5 had complexed 160 µg hGH/200 µg EB01 thrice weekly for 1 or 4 weeks respectively; and Group 6 had daily injections of the said complex for 4 weeks. Groups 5 and 6 grew by 31% and 37% more than PBS injected controls (Figure 2). A significant weight gain effect of hGH-EB01 complexes was apparent as early as 48 hours after administration (Table 2).

Animals receiving 10, 40 or 160 µg of hGH in the presence of EB01 demonstrated a significant weight gain increment when compared with hGH only.

TABLE 2
Short-term weight gains in juvenile BALB/c mice injected with
various doses of hGH complexed with EB01 antibody

| hGH µg | hGH-EB01 complex (160/200 µg) | Relative weight gain % ± SD | p (significance) |
|---|---|---|---|
| — | – | 13.0 ± 11.0 | |
| 10 | – | 16.0 ± 12.0 | <0.050 |
| | + | 27.0 ± 6.0 | |
| 40 | – | 16.0 ± 11.0 | <0.050 |
| | + | 29.0 ± 8.0 | |
| 160 | – | 13.0 ± 13.0 | <0.025 |
| | + | 29.0 ± 11.0 | |

Three week old mice ($W_{to}=8.0\pm1.0$) were injected at time 0 and 24 h. Weight gains, expressed as % of body weight, were determined at 48 h.

Example D
Potentiation of the lactogenic activity of hGH

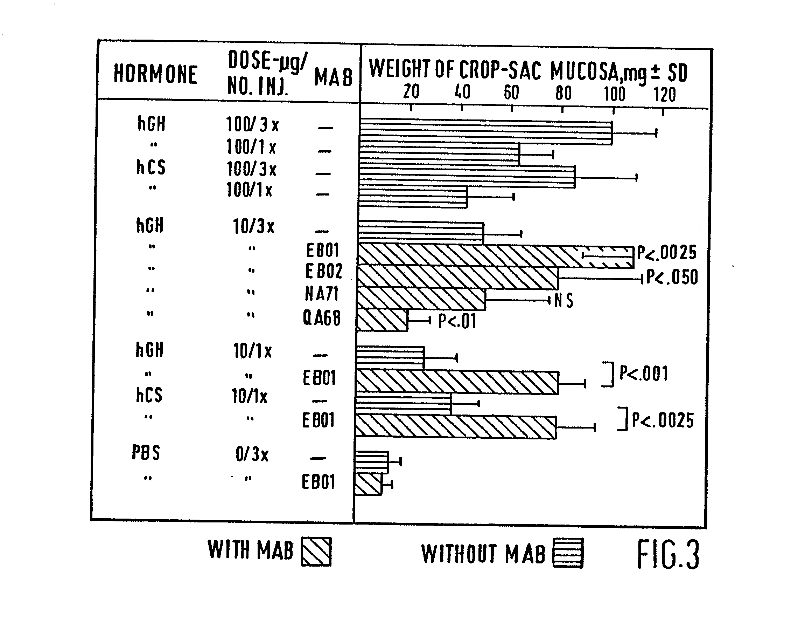

Human growth hormone produces significant lactogenic activity as measured by its effects on pigeon crop-sac or mammary tissue *in vivo* and by its ability to displace [125]I-hPRL from binding to mammary gland receptors. The pigeon crop-sac bioassay procedure measures the lactogenic activity of hormones and is analogous to other mammotropic assays involving either mammary gland or corpus luteum of rats or mice. Hormones, complexes or control solutions were administered (0.1 ml) from coded vials intradermally adjacent to each hemicrop, there being five birds in each group. The injection protocol was either one administration on day 1 only ($2\times10^4$ $ABT_{50}$) or three over 36 hours (each of $2\times10^3$ $ABT_{50}$) or combined with two further injections on day 2. Birds were killed on day 3 and the wet weight of the crop-sac mucosa of 2.5 cm diameter was determined (Nicoll, 1967). By administering the complex or free hormone intradermally, adjacent to each of the two individual hemicrops, the potentiation effect has been examined under conditions which excluded systemic hormone distribution. The weight of the crop-sac mucosa following the injection of 100 µg of hGH in three doses was about 100 mg, whereas the control (PBS or antibody only treated) mucosa weighed 10—13 mg (Figure 3).

Treatment with 10 µg of hormone alone produced a mucosa of 48 mg, but in the presence of EB01 or EB02 the mucosal weight increased to 108 mg and 80 mg respectively. As in dwarf mice, NA71 was without potentiating activity whereas QA68 significantly depressed the mucosal secretion. Furthermore, EB01 potentiated the lactogenic effects proportionally by the same extent whether the hormone was administered in one or three doses. However, EB01 failed to potentiate the lactogenic effect of saturating doses of hGH. Since EB01 binds equally to hCS, we also examined the potentiation of the lactogenic activity of this hormone. The results show that antibody complexes with 10 µg hCS had doubled the weight of the crop-sac mucosa in comparison with controls receiving the hormone alone.

Example E
Potentiation of growth in marmosets

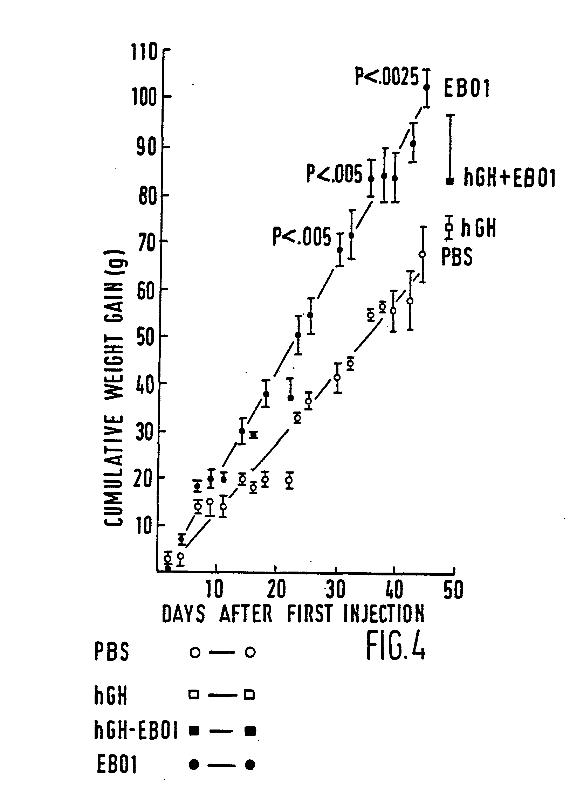

The potentiation effect in either murine growth or pigeon crop-sac responses was dependent on the administration of exogenous hGH since none of the antibodies described here cross-reacted with the rodent growth hormone. However, we discovered that EB01 antibody did bind to marmoset growth hormone when tested by immunoblot assay. This observation enabled the assessment of EB01 potentiation of marmoset growth in the absence of any exogenously administered hormone (Figure 4). Sixteen animals, randomised on a weight basis, were divided into four groups receiving 0.4 mg hGH only, hGH (0.4 mg)+EB01 (2 mg), EB01 (2 mg) only or PBS three times per week. Two sister animals had to be removed from the experiment after 1 week due to continuous weight loss. Animals treated for 44 days with PBS increased their weight by 68 g, whereas the group receiving EB01 only demonstrated a mean weight increase of about 103 g. Groups receiving hormone only or complex had weight gains intermediate to those observed with EB01 only and the control group. The relative weight gain (% over initial body weight) of animals receiving antibody was 89% whilst the control group increased their body weights by only 61%.

Despite continuous administration of heterologous antibody to the marmosets for 6 weeks, side effects, possibly of anaphylactic origin, have so far not been observed.

Example F

Further pigeon crop sac assay

The pigeon crop sac assay of Example D was repeated with 1 µg of highly purified (prep. L) or 3 µg of QB01-MAB affinity purified (prep. R) hPRL alone or in complex with a constant dose, 2500 $ABT_{50}$ of monoclonal antibodies. The preparation of QB01 antibodies was analogous to that described in Example 1 above and has been published (Ivanyi & Davies, 1981).

The results, given in Table 3, show strong potentiation for antibody QB01, for both preparation of hPRL. The figures represent mean values for six crops per group≡one standard deviation.

TABLE 3

| Antibody | Weight in crop sac (mg) | |
|---|---|---|
| | Prepn. L | Prepn. R |
| QB01 | 68.0±30.5 | 75.0±18.0 |
| Control A (hPRL alone) | 22.5±14.5 | 22.0±11.0 |
| Control B (uninjected) | 19.5±5.0 | |

Example G

Dwarf mouse body growth and composition

Dwarf (dw/dw) mice were divided into three groups of eighteen and fed on 100%, 75% or 50% of the usual *ad lib* consumption for 10 days. Within each diet, six mice were treated with saline, six with hGH (40 milliunits) and six with hGH/EB01 ($10^4$ $ABT_{50}$ of antibody) complex. The mice were assessed for overall growth ($^{35}SO_4$ uptake into intercostal cartilage, sulphate injected at day nine), fat content, weight change and length of tail. The results are presented in Tables 4 to 7.

It is apparent that the use of a hormone/antibody complex can compensate for a reduced diet and can also reduce the proportion of fat in an animal by preferentially causing growth of muscle, the latter advantage being even more marked with a reduced diet.

TABLE 4
Sulphate uptake into intercostal cartilage

| | Diet | | |
|---|---|---|---|
| | 100% | 75% | 50% |
| Treatment | | | |
| Saline | 2393±315 | 695±76 | 540±57 |
| hGH | 5569±209 | 4492±317 | 2958±202 |
| hGH+EB01 | 7956±865 | 5531±691 | 4840±668 |

The results are expressed as disintegrations per minute per milligram of tissue; mean ±S.E.

TABLE 5
Fat content

| | Diet | | |
|---|---|---|---|
| | 100% | 75% | 50% |
| Saline | 2533±83 | 1502±304 | 1082±89 |
| hGH | 1857±130 | 1173±127 | 631±152 |
| hGH+EB01 | 1164±99 | 733±51 | 347±91 |

Figures are amounts of fat in milligrams.

TABLE 6
Weight change as a % of initial weight (initial weight=100%)

| | Diet | | |
|---|---|---|---|
| | 100% | 75% | 50% |
| Treatment | | | |
| Saline | 104.67±0.95 | 94.5±1.63 | 87.83±1.14 |
| hGH | 119.67±1.94 | 107.5±1.61 | 91.67±0.8 |
| hGH+EB01 | 127.17±2.12 | 111.67±0.88 | 95.2±2.52 |

Figure expressed as mean ±S.E.

TABLE 7
Longitudinal growth of tail (increase in mm)

| | Diet | | |
|---|---|---|---|
| | 100% | 75% | 50% |
| Treatment | | | |
| Saline | 0.96±0.24 | 0.5±0.19 | 0.71±0.36 |
| hGH | 3.33±0.3 | 3.29±0.47 | 2.04±0.28 |
| hGH+EB01 | 5.21±0.39 | 4.96±0.31 | 4.05±0.16 |

Figure expressed as mean ±S.E.

Example H

Growth in normal mice

The experiment of Example C was repeated, with the assay being for $^{35}SO_4$ uptake into intercostal cartilage rather than weight gain. Mice aged 4 weeks (weight 10 g), 6 weeks (14 g) and 9 weeks (19 g) were used and injected subcutaneously with 0.1 ml of either saline, hGH (100 µg) or hGH-EB01 complexes (100 µg/$10^4$ ABT$_{50}$) two days before administration of $^{35}SO_4$. Cartilage was removed 24 h later. The results are given in Table 8 and shown significantly increased growth with the complexes (p<0.0005 for the oldest mice).

TABLE 8

| | Mice age | | |
|---|---|---|---|
| Treatment | 4 weeks | 6 weeks | 9 weeks |
| Saline | 1686±282 | 1176±85 | 505±32 |
| hGH | 1621±158 | 1330±83 | 605±54 |
| hGH/EB01 | 2431±307 | 1738±191 | 1071±85 |

Units: counts/min/mg. cartilage.

Example I

Use of polyclonal antibodies of reduced specificity

A 7 K (7000 Daltons) fragment was cleaved from the C-terminal of hGH with subtilisin followed by chromatography under denaturing conditions (Aston & Ivanyi 1983). Two mice were each injected with 50 µg of the fragment emulsified with Freund's complete adjuvant but without a carrier and 21 days later received a further 50 µg without any adjuvant. Serum was taken 10 days after the second challenge, complexed with hGH (10 µg) and injected into dwarf mice. The subsequent growth of mice was assayed by measuring $^{35}SO_4$ uptake as above. The results (Table 9) show that the polyclonal antiserum potentiated growth.

TABLE 9

| Treatment | $^{35}SO_4$ uptake (c.p.m./mg.cartilage) |
|---|---|
| Saline | 500±50 |
| hGH (10 µg) | 1365±146 |
| hGH plus antiserum I | 4425±703 |
| hGH plus antiserum II | 3272±471 |

References

Aston, R. and Ivanyi, J. The EMBO Journal 2 493—497 (1983).

Fisher, R. A. and F. Yates (Ed.) "Statistical tables for biological, agricultural and medical research" Oliver ° Boyd (1957).

Herbai, G. Acta. Physiol. Scandinavica 80 (1970) 470—491.

Hughes, P. C. R. and Tanner, J. M. J. Anatomy 106 (1970) 349—370.

Ivanyi J. In: Monoclonal Hybridoma Antibodies: Techniques and Applications (Edited by Hussell, J. G. R.) CRC Press, Cleveland, Ohio (1982) pp. 59—79.

Ivanyi J. Mol. Immunol. 19 (1982) 1611—1618.

Ivanyi J. and P. Davis. Mol. Immunol. 17 (1980) 287—290.

Ivanyi J. and P. Davis. Protides of the Biological Fluids 29th Colloquium 1981.

Ed. H. Peeters Pergamon Press, Oxford and New York, 1982, 855—860.

Linde, S., B. Hausen, A. Lernmark. Analyt Biochem 107 (1980) 165—176.

Nicoll, C. S. Endocrinology 80 641—655 (1967).

Example J

Potentiation of ovine growth hormone activity

Monoclonal antibodies to ovine (i.e. sheep) growth hormone (oGH) were prepared in an analogous way to the method of Example 1, and are available from the Department of Experimental Immunobiology, Wellcome Research Laboratories, Langley Court, Beckenham, Kent, BR3 3BS, UK. Dwarf mice, which respond to oGH, were divided into groups of six and treated with 50 µg of the hormone, either alone or complexed with one of four such antibodies, on two consecutive days prior to injection with $^{35}SO_4$. Intercostal cartilage was removed 24 hours later, dissolved in formic acid and the radioactivity counted. The results are given in Table 1:

TABLE 10

| Treatment | MAB titre | Uptake of $^{35}SO_4$ (mean±S.E.;cpm/mg tissue) |
|---|---|---|
| oGH alone | — | 1411±261 |
| oGH+1D11 H9 | $1 \times 10^{-4}$ | 5871±1339 |
| oGH+4B62 D9 | $3.2 \times 10^{-4}$ | 4323±671 |
| oGH+2B11 | $4.2 \times 10^{-3}$ | 3408±642 |
| oGH+3B11 | $5.6 \times 10^{-2}$ | 3434±719 |
| Saline | — | 557±79 |

Example K
Potentiation of growth in sheep

Groups of two sheep (mean weight 17 kg) were treated with differing doses of anti-oGH antibody 2B11 (see Example J) or, as a control, mouse globulin, on two consecutive days before intraperitoneal injection of $^{35}SO_4$ (146 µCi/kg). Quadruplicate samples of intercostal cartilage were removed from each site 24 hours later and analysed as above. The results are in given Table 11:

TABLE 11

| Treatment | Incorporation of $^{35}SO_4$ (mean ±S.D.; cpm/10 mg cartilage) |
|---|---|
| $10^6$ ABT$_{50}$ 2B11 | 3094±630 |
| $0.2×10^6$ ABT$_{50}$ 2B11 | 5168±24 |
| $0.04×10^6$ ABT$_{50}$ 2B11· | 2373±183 |
| Control | 2084±771 |

These results, which are highly significant by variance analysis, show that formulations in accordance with the invention can potentiate the action of endogenous GH in an economically important species.

Example L
Potentiation of growth in sheep

Example K was repeated additionally using a different monoclonal antibody, 1D11 H9, and groups of five sheep, mean weight 24 kg. The results are given in Table 12:

TABLE 12

| Treatment | $^{35}SO_4$ Uptake (mean ±S.E.; cpm/10 mg) |
|---|---|
| $8.8×10^5$ ABT$_{50}$ 2B11 | 3315±560 |
| $2×10^6$ ABT$_{50}$ 1D11 H9 | 2818±343 |
| control immunoglobulin | 1908±299 |

Significantly ($p<0.05$) increased growth is seen with the MAB-treated groups.

Example M
Potentiation of thyroid stimulating hormone (TSH) activity

TSH is a glycoprotein produced by the pituitary gland and activating the thyroid gland in vertebrates. A deficiency of TSH causes involution of the thyroid gland and flattening of the epithelium. In humans, such a deficiency can be responsible for cretinism, simple goitre and the panoply of abnormal conditions known collectively as myxedema. It may be treated with iodine compounds or thyroid gland extracts. Dwarf mice are hypopituitary and the thyroid gland is involuted. Treatment with TSH raises the serum $T_4$ levels and causes some histological repair of the thyroid.

A monoclonal antibody (GC73) to TSH was prepared analogously to those of Example 1 above and is available from the same address as in Example J. GC73 is specific for the β-chain of TSH. Dwarf mice were randomly divided into groups of five and treated accordingly to the regimes of Table 13 on five consecutive days before analysis of the serum for $T_4$ level by radioimmunoassay, and microscopic histological inspection of thyroid tissue, fixed in 10% formalin in saline solution and then embedded in wax or plastic. The $T_4$ data are given in Table 13; the results were confirmed by microscopic examination.

TABLE 13

| Treatment | $T_4$ level (mean $\pm$ S.E.) |
|---|---|
| 0.1 international units TSH | $62.8 \pm 3.7$ |
| 0.05 units TSH | $36.4 \pm 6.3$ |
| 0.1 units TSH + $10^4$ $ABT_{50}$ GC73 | $116.2 \pm 5.3$ |
| 0.05 units TSH + $10^4$ $ABT_{50}$ GC73 | $72.2 \pm 11.75$ |
| Saline | less than 5 |
| $10^4$ $ABT_{50}$ GC73 only | less than 5 |

## Claims

1. An injectable formulation comprising a pharmaceutically acceptable sterile injectable carrier and antibodies to a hormone other than insulin and EGF, the epitope specificity of at least some of the antibodies being so chosen that the formulation will potentiate, when administered to a vertebrate, the administration of the hormone in that vertebrate.

2. An injectable formulation comprising a pharmaceutically acceptable sterile injectable carrier and complexes of (a) a hormone other than insulin and EGF and (b) at least one type of antibody to that hormone, the epitope specificity of at least some of the antibodies being so chosen that the formulation will potentiate, when administered to a vertebrate, the administration of the hormone in that vertebrate.

3. A formulation according to Claim 1 or 2, in which the hormone is growth hormone, prolactin, chorionic somatomammotropin or thyroid stimulating hormone.

4. A formulation according to Claim 3, wherein the hormone is growth hormone.

5. A formulation according to any one of the preceding claims, wherein the antibody is a monoclonal antibody.

6. A formulation according to Claim 4 or Claim 5, wherein the antibody is a monoclonal antibody having substantially the same epitope specificity as EB01 or EB02.

7. A method of potentiating hormone administration in a "normal" vertebrate (as herein defined) by administering to the vertebrate by injection a formulation according to any one of the preceding claims.

8. A method of increasing a hormone-regulated response of a "normal" vertebrate (as herein defined) by administering to the vertebrate a preparation comprising at least one pre-selected antigenic "fragment" (as herein defined) of a hormone other than insulin or EGF, optionally in combination with an adjuvant, such as to raise potentiating antibodies to the hormone in the vertebrate.

9. A method according to Claim 8, wherein the hormone is growth hormone, prolactin, chorionic somatomammotropin or thyroid stimulating hormone.

10. A method according to Claim 7 or Claim 8, wherein the response is regulated by growth hormone.

11. A formulation comprising a potentiating antigenic "fragment" (as herein defined) of a hormone other than insulin or EGF, together with an immunological carrier and/or an adjuvant, in combination with a pharmaceutically acceptable carrier.

12. A formulation according to Claim 11, wherein the hormone is selected from the group consisting of growth hormone, prolactin, chorionic somatomammotropin and thyroid stimulating hormone.

13. A process for preparing a formulation according to Claim 1 comprising the steps of
(a) preparing a monoclonal antibody of restricted specificity to the said hormone
(b) determining whether the antibody from step (a) potentiates the activity of the hormone and, if it does,
(c) bringing the antibody into association with a sterile injectable pharmaceutically acceptable carrier.

14. A process according to Claim 13 for preparing a formulation according to Claim 2, wherein, after step (b) and before step (c), the antibody is complexed with the said hormone.

## Patentansprüche

1. Injizierbare Formulierung umfassend einen pharmazeutisch annehmbaren sterilen injizierbaren Träger und Antikörper auf ein anderes Hormon als Insulin und EGF, wobei die Epitopspezifität mindestens einiger der Antikörper so gewählt wird, daß die Formulierung bei Verabreichung an ein Wirbeltier die

EP 0 137 234 B1

Verabreichung des Hormons in diesem Wirbeltier verstärkt.

2. Injizierbare Formulierung umfassend einen pharmazeutisch annehmbaren sterilen injizierbaren Träger und Komplexe (a) eines anderen Hormons als Insulin und EGF und (b) mindestens einer Art von Antikörper auf dieses Hormon, wobei die Epitopspezifität mindestens einiger der Antikörper so gewählt wird, daß die Formulierung bei Verabreichung an ein Wirbeltier der Verabreichung des Hormons in diesem Wirbeltier verstärkt.

3. Formulierung nach Anspruch 1 oder 2, in der das Hormon Wachstumshormon, Prolaktin, Chorion-Somatomammotropin oder schilddrüsenstimulierendes Hormon ist.

4. Formulierung nach Anspruch 3, worin das Hormon Wachstumshormon ist.

5. Formulierung nach einem der vorhergehenden Ansprüche, worin der Antikörper monoklonaler Antikörper ist.

6. Formulierung nach Anspruch 4 oder 5, worin der Antikörper ein monoklonaler Antikörper mit im wesentlichen der gleichen Epitopspezifität wie EB01 oder EB02 ist.

7. Verfahren zum Verstärken der Hormonverabreichung in einem "normalen" Wirbeltier (wie hier definiert) durch Verabreichung einer Formulierung nach einem vorhergehenden Ansprüche mittels Injektion an das Wirbeltier.

8. Verfahren zum Erhöhen einer hormonregulierten Reaktion eines "normalen" Wirbeltieres (wie hier definiert) durch Verabreichen eines Präparates umfassend mindestens ein vorgewähltes antigenes "Fragment" (wie hier definiert) eines anderen Hormons als Insulin oder EGF, gegebenenfalls in Kombination mit einem Adjuvans, an das Wirbeltier, derart, daß verstärkende Antikörper auf das Hormon im Wirbeltier gebildet werden.

9. Verfahren nach Anspruch 8, worin das Hormon Wachstumshormon, Prolaktin, Chorion-Somatomammotropin oder schilddrüsenstimulierendes Hormon ist.

10. Verfahren nach Anspruch 7 oder 8, worin die Reaktion durch Wachstumshormon reguliert wird.

11. Formulierung umfassend ein verstärkendes antigenes "Fragment" (wie hier definiert) eines anderen Hormons als Insulin oder EGF zusammen mit einem immunologischen Träger und/oder einem Adjuvans in Kombination mit einem pharmazeutisch annehmbaren Träger.

12 Formulierung nach Anspruch 11, worin das Hormon ausgewählt ist aus der Gruppe bestehend aus Wachstumshormon, Prolaktin, Chorion-Somatomammotropin und schilddrüsenstimulierendem Hormon.

13. Verfahren zum Herstellen einer Formulierung nach Anspruch 11 umfassend folgende Schritte:
(a) Herstellen eines monoklonalen Antikörpers mit beschränkter Spezifität auf dieses Hormon,
(b) Bestimmen, ob der Antikörper von Schritt (a) die Aktivität des Hormons verstärkt, und, wenn ja,
(c) Vereinigen des Antikörpers mit einem sterilen injizierbaren pharmazeutisch annehmbaren Träger.

14. Verfahren nach Anspruch 13 zum Herstellen einer Formulierung nach Anspruch 2, worin nach Schritt (b) und vor Schritt (c) der Antikörper mit diesem Hormon in einen Komplex gebracht wird.

## Revendications

1. Composition injectable comprenant un excipient injectable stérile pharmaceutiquement acceptable et des anticorps à l'égard d'une hormone autre que l'insuline et l'EGF, la spécificité d'épitope d'au moins certains des anticorps étant choisie de façon que la composition potentialise, lorsqu'elle est administrée à un vertébré, l'administration de l'hormone à ce vertébré.

2. Composition injectable comprenant un excipient injectable stérile pharmaceutiquement acceptable et des complexes (a) d'une hormone autre que l'insuline et l'EGF et (b) d'au moins un type d'anticorps contre cette hormone, la spécificité d'épitope d'au moins certains des anticorps étant choisie de façon que la composition potentialise, lorsqu'elle est administrée à un vertébré, l'administration de l'hormone à ce vertébré.

3. Composition suivant la revendication 1 ou 2, dans lauqelle l'hormone est l'hormone de croissance, la prolactine, la somatomammotropine chorionique ou l'hormone thyréotrope.

4. Composition suivant la revendication 3, dans laquelle l'hormone est l'hormone de croissance.

5. Composition suivant l'une quelconque des revendications précédentes, dans laquelle l'anticorps est un anticorps monoclonal.

6. Composition suivant la revendication 4 ou 5, dans laquelle l'anticorps est un anticorps monoclonal ayant sensiblement la même spécificité d'épitope que EB01 ou EB02.

7. Procédé pour potentialiser une administration d'hormone chez un vertébré "normal" (tel que défini ici) en administrant à ce vertébré par injection une composition suivant l'une quelconque des revendications précédentes.

8. Procédé pour augmenter la réponse à régulation hormonale d'un vertébré "normal" (tel que défini ici) en administrant à ce vertébré une préparation comprenant au moins un "fragment" antigénique présélectionné (tel que défini ici) d'une hormone autre que l'insuline ou l'EGF, éventuellement en combinaison avec adjuvant, de façon à susciter des anticorps contre l'hormone potentialisants chez ce vertébré.

9. Procédé suivant la revendication 8, dans lequel l'hormone est l'hormone de croissance, la prolactine, la somatomammotropine chorionique ou l'hormone thyréotrope.

**EP 0 137 234 B1**

10. Procédé suivant la revendication 7 ou 8, dans lequel la réponse est régulée par l'hormone de croissance.

11. Composition comprenant un "fragment" antigénique potentialisant (tel que défini ici) d'une hormone autre que l'insuline ou l'EGF, conjointement avec un excipient immunologique et/ou un adjuvant, en combinaison avec un excipient pharmaceutiquement acceptable.

12. Composition suivant la revendication 11, dans laquelle l'hormone est choisie dans la classe formée par l'hormone de croissance, la prolactine, la somatomammotropine chorionique et l'hormone thyréotrope.

13. Procédé pour préparer une composition suivant la revendication 1, qui comprend les stades:

(a) de préparation d'un anticorps monoclonal ayant une spécificité réduite contre l'hormone,

(b) la détermination du fait que l'anticorps du stade (a) potentialise ou non l'activité de l'hormone et s'il le fait,

(c) la mise de l'anticorps en association avec un excipient injectable stérile pharmaceutiquement acceptable.

14. Procédé suivant la revendication 13 pour la préparation d'une composition suivant la revendication 2, dans lequel, après le stade (b) et avant le stade (c), l'anticorps est complexé avec l'hormone.

FIG.1

x-x  PBS(control)
o-o  hGH
●-●  hGH-EB01 (10μg/200μg)
■-■     ''      (160μg/200μg)

FIG. 2

FIG.3

WITH MAB ▨    WITHOUT MAB ▦

FIG.4